Europäisches Patentamt

(19) European Patent Office    (11) Publication number: **0 048 202**

Office européen des brevets    **A2**

(12)                    # EUROPEAN PATENT APPLICATION

(21) Application number: **81401408.0**    (51) Int. Cl.³: **C 12 N 5/00**
                                                    **C 12 R 1/91**

(22) Date of filing: **10.09.81**

(30) Priority: **11.09.80 US 185054**

(43) Date of publication of application:
**24.03.82 Bulletin 82/12**

(84) Designated Contracting States:
**AT BE CH DE FR GB IT LI LU NL SE**

(71) Applicant: **MERCK & CO. INC.**
**126, East Lincoln Avenue P.O. Box 2000**
**Rahway, New Jersey 07065(US)**

(72) Inventor: **Schlabach, Abner J.**
**222 Blue School Road**
**Perkasie Pennsylvania 18944(US)**

(74) Representative: **Schrimpf, Robert et al,**
**Cabinet Regimbeau 26, Avenue Kléber**
**F-75116 Paris(FR)**

(54) Method of preparing a mycoplasma-free hepatoma cell line.

㊼ A hepatoma cell line which sheds HBsAg but which is free of mycoplasma is obtained by injecting immuno-suppressed mice with cells from the hepatoma cell line and isolating cells from tumors which develop in the mice.

EP 0 048 202 A2

- 1 -

METHOD OF PREPARING A
MYCOPLASMA-FREE HEPATOMA CELL LINE

BACKGROUND OF THE INVENTION

Human hepatoma cell lines which shed hepatitis B surface antigen (HBsAg) are known. See for example, Alexander et al., South African J. Med. Sci., 41(2): 89-98, (1976); MacNab et al., Br. J. Cancer (1976) 34: 509-515; and Alexander et al., South African Med. J. (1976) pages 2124-2128. A problem with such cell lines, however, is that they contain mycoplasma. Mycoplasma are undesirable as they are a contaminant to tissue culture and also because they may affect cell growth and yields.

OBJECTS OF THE INVENTION

It is, accordingly, an object of the present invention to provide a method for irradicating the mycoplasma contamination from a hepatoma cell line. Another object is to provide a mycoplasma free hepatoma cell line. These and other objects of the present invention will become apparent from the following description.

SUMMARY OF THE INVENTION

A hepatoma cell line which sheds HBsAg and which is free of mycoplasma is obtained by injecting an immuno-suppresed mammalian species with cells from the hepatoma cell line and thereafter isolating cells from tumors which develop subsequently in the mammalian species.

DETAILED DESCRIPTION

Any human hepatoma cell line which sheds HBsAg may be used in carrying out the present invention. A specific and preferred example of such a cell line is Alexander cells, a culture of which has been deposited with American Type Culture Collection, Bethesda, Maryland, U.S.A., under accession number CCL 8024.

According to the present invention, cells from the human hepatoma cell line which sheds HBsAg are injected intradermally or subcutaneously (s.c.) into an immunosuppressed mammalian species and cells from a resulting tumor are grown in cell culture to form a mycoplasma-free cell line. While any mammalian species may be employed, preferred species are readily obtainable laboratory animals which are small in size, easily handled, and economical. Examples of preferred mammalian species are guinea pigs, hamsters, mice, rabbits and rats.

The mammalian species selected has either a naturally or artificially induced defective immune system. Thus, the mammalian species may be a genetically immuno-deficient animal, e.g. nude (nu/nu) mice. Alternatively the immuno-suppression may be effected by radiation, e.g. by x-ray or cobalt-60, or chemically, e.g. by use of cyclophosphamide plus anti-thymocyte serum (ATS).

The isolated tumor cells may be grown in cell culture under conditions described in the article by MacNab et al., op. cit., and in the articles by Alexander et al., op. cit.  The resulting cell line is free of mycoplasma but is otherwise morphologically identical to the original HBsAg-shedding human hepatoma cell line.

The following examples illustrate the present invention without, however, limiting the same thereto.

## EXAMPLE 1

Cell culture fluids from a human hepatoma cell line (Alexander cells PLC/PRF/5, ATCC CCL 8024) culture are placed directly on agar medium with and without filtering through a 0.22 micron filter. In addition, culture fluids are incubated (37°C) in a mycoplasma enrichment broth for 10 days and then plated onto agar. The agar cultures are incubated at 37°C both aerobically and anareobically in a moist atmosphere. After at least 10 days incubation, the cultures are examined for the presence of mycoplasma based on microscopic observation of colony morphology, resistance of the colonies to scraping and the reaction of the colonies to dienes stain. All cell fluids are found to be positive for mycoplasma.

## EXAMPLE 2

Alexander cells are grown in plastic flasks using Eagle's Minimum Essential Medium (EMEM), supplemented with 10% fetal calf serum, L-glutamine (2mM), and Neomycin (50 μg/ml). The cells are cultured in the presence of 10 μg/ml of tetracycline, removed from the cultured flasks with 0.25% trypsin, centrifuged, and resuspended at $10^7$ cells/ml. Five hamsters are anesthetized and 0.1 ml of cell suspension ($10^6$ cells) is injected into the tissue of each cheek pouch to form a bleb. Fifteen mice are injected intradermally on their abdominal surface with the same amount ($10^6$ cells in 0.1 ml). The mice are injected subcutaneously on day -1 with 0.4 ml of anti-thymocyte serum (ATS) and 0.2 ml ATS daily for the remainder of the experiment except for days 5, 6, 12 and 13. On days 3, 4, 8, and 9 each hamster is given 5 μg/animal and each

mouse is given 1.25 μg of oral tetracycline. On day 16, all of the animals are examined for tumors. All are negative. This experiment shows that ATS alone is inadequate to induce complete immuno-suppression.

EXAMPLE 3

Twelve mice are each given 3.0 mg of cyclophosphamide intraperitoneally (i.p.), and 75 minutes later are intradermally injected with $4 \times 10^6$ hepatoma cells on the abdominal surface. The mice are given ATS according to the following schedule.

Day

| 1 | 0.2 ml, i.p. |
| 3 | 0.4 ml, s.c, plus 0.5 ml, i.p. |
| 6 | 0.5 ml, i.p. |
| 7 | 0.5 ml, i.p. |

On day 8, three mice have apparent tumors. They are given 1.0 ml, i.p. in 2 doses 6 hours apart on both day 8 and day 9. On day 14 all three mice still have apparent tumors. The tumor from one mouse is excised aseptically, dispersed by mincing with a scissors, and trypsinized successively for 15 and 20 minutes. The cells are pooled and planted into plastic flasks and cultured under usual culture conditions (5% $CO_2$ and 95% relative humidity) with Minimum Essential Medium, Eagle (EMEM), 10% fetal calf serum (fcs) and 50 μg/ml neomycin. No living cells are detected in the cultures examined over the next 6 days.

The remaining 2 mice are treated as follows:

Day

| 14 | 0.5 ml ATS, i.p. |
| 15 | 0.5 ml ATS, i.p. |
| 16 | 0.5 ml ATS, i.p. |
| 17 | 0.75 ml ATS, i.p. |
| 20 | 0.75 ml ATS, i.p. |

On day 20, one tumor appears to be regressing; the other is getting larger.

Day
21   0.75 ml ATS i.p.
22   0.75 ml ATS i.p.

On day 23, the prominent tumor measures about 1 cm in diameter and protrudes about 4 mm from the abdomen. It is excised aseptically, dispersed by mincing with a scissors, and trypsinized twice for 30 minutes. The cells dispersed from the two trypsinizations are separately planted in plastic flasks in growth medium (EMEM, 10% fcs, 50 µg/ml neomycin). Both cultures grow into monolayers. The cells from the first trypsinization are subsequently lost to mold contamination. The cells from the second trypsinization continue to grow well and are expanded into multiple cultures. No mycoplasma is detected in any of the cultures.

EXAMPLE 4

Culture of tissue fluids from Alexander cell cultures on PPLO agar medium results in easily detected mycoplasma colonies after about 8 days, whether incubated aerobically or anaerobically at 37°C. The cell culture fluids derived from the third mouse tumor in Example 3 are cultured repeatedly in parallel to Alexander cell culture fluids (as controls) and are consistently negative for mycoplasma colonies.

WHAT IS CLAIMED IS:

1. A method of preparing a hepatoma cell line which sheds HBsAg but which is free of mycoplasma which method comprises injecting cells from a hepatoma cell line which sheds HBsAg into an immuno-suppressed mammalian species, isolating hepatoma cells from tumors which develop in the mice, and growing the isolated hepatoma cells in cell culture.

2. A method according to claim 1 wherein the hepatoma cell line is derived from Alexander cells.

3. A method according to claim 1 wherein the mammalian species is a readily obtainable laboratory species.

4. A method according to claim 3 wherein the species is guinea pig, hamster, mouse, rabbit or rat.

5. A method according to claim 1 wherein the mammalian species is genetically immuno-deficient.

6. A method according to claim 1 wherein the mammalian species is artificially immuno-suppressed.

7. A method according to claim 6 wherein the immuno-suppression is effected by radiation.

8. A method according to claim 6 wherein the immuno-suppression is effected chemically.

9. A method according to claim 8 wherein the immuno-suppression is effected by cyclophosphamide and ATS.

10. A human hepatoma cell line which sheds HBsAg which has been rendered free of mycoplasma but which is morphologically identical to the original mycoplasma-containing human hepatoma cell line.